# EUROPEAN PATENT APPLICATION

(11) **EP 0 767 377 A1**
(43) Date of publication of application: **09.04.1997**
(21) Application number: 95250244.1
(22) Date of filing: 04.10.1995
(51) Int. Cl.: G01N 33/569, G01N 33/564, G01N 33/58, G01N 33/543

(54) **Diagnosis of pneumococcal infection by measurement of antigen specific immune complexes**

(71) Applicant: Dr. Fooke Laboratorien GmbH, 41469 Neuss (DE)
(72) Inventor: Fooke, Margrit Dr., D-41469 Neuss (DE)
(74) Representative: Wehlan, Helmut, Dr.

(57) **Abstract**

The invention relates to the diagnosis of pneumococcal infection by the detection and quantitation of pneumococcal-specific antigen containing immune complexes, especially by the binding of the complexes to conglutinin or to substances with properties similar to conglutinin wheter by solid phase or liquid phase attachment.

## Description

This invention relates to the diagnosis of pneumococcal infection. The responsible organism, Streptococcus pneumoniae, is the commonest cause of pneumonia in man. This organism, often call pneumococcus, also causes other serious infections such as meningitis, otitis media, sinusitis and exacerbations of chronic bronchitis.

The diagnosis of pneumococcal infection is difficult and is often attempted by trying to culture the organism from specimens of blood, sputum or other secretions, swabs of pus or pleural fluid. Using any or all of these methods, the success rate is rarely greater than 30%. An alternative approach has been serological examination in which antigen or antibody to the antigen is measured in the patients' sera. Antigen is a foreign substance, in this case specific to the pneumococcus, and antibody is a protein produced by the human host in response to infection by the organism and which is directed towards the antigen or antigens. Measurements of pneumococcal antigens or antibody to these antigens in the blood of infected patients have been used as diagnostic tests in pneumococcal infections but have not been very successful.

A problem with serological diagnosis is that the antigen or antigens liberated by the pneumococcus combine with the antibody formed by the host to produce immune complexes which exist in the blood for variable periods of time. When antigen or antibody is complexed, they are not measurable by conventional methods and measurement requires a more sophisticated approach which can recognise these complexes and provide a method for analysing and measuring of the complex components.

Many methods of immune complex detection have been described but most of these are descriptions of antigen non-specific immune complex detection and are of no use for the purpose of diagnosis of pneumococcal infection. Antigen - specific methods have been described (Mellencamp et al. 1987) but the technique used has been cumbersome, expensive and most importantly, non-quantitative. The latter method has relied on detection without quantitation of the pneumococcal antigen in the isolated immune complexes. Further, it is not possible with tests such as those described by Mellencamp et al. (1987) to discriminate between different types of pneumococcal infection, and this method is therefore of limited benefit in patient care.

Other, more appropriate methods of antigen - specific immune complex detection have been described. For example, US Patent No. 4,548,909 - "A Method of Diagnosis" describes the use of a conglutinin - based procedure for the detection of immune complexes followed by identification of the antigen component of the complexes for the diagnosis of infective diseases. Similarly, Manca et al. (1988), Bo-Hai W, and Shu-Rong Y (1987), respectively demonstrate the detection of the antigens of Schistosoma haematobium and Coxiella burnetii in immune complexes by using conglutinin.

Conglutinin, as a binding agent for immune complexes, seems to be a suitable agent. However, these previous publications do not solve the problems posed by pneumococcal infection in man. None of the cited examples are pertinent to this infection. Further, US Patent No. 4,548,909 does not describe a method of quantitation of antigen within the complexes. Manca et al. (1988), similarly, demonstrate only the detection of Schistosomal antigen and not its measurement. This research is relevant only for the infection described and has little significance in respect of pneumococcal infection. Similarly, the publication of Bo-Hai and Shu-Rong (1987) refers to an experimental model of Coxiella burnetii infection in guinea-pigs which is of no relevance to the diagnosis of pneumococcal infection in man.

In the present invention a method is described whereby human pneumococcal - containing complexes can be identified and the antigen component of pneumococcal origin in the complex can be quantified using conglutinin as the immune complex binding agent. According to the present invention there is provided a method of diagnosis of pneumococcal infection comprising a material for attachment to an immune complex, obtaining an extract from a living body, treating the body extract with said material thereby to attach any immune complex present in the extract to said material, and detecting and quantitating the attached immune complexed antigen using anti-antigen sera specific for common or serotype - specific antigens of Strep. pneumoniae. The presence of absence of reaction between each reagent will identify the presence or absence of the pneumococcal antigen sought.

By using the method of this invention it is possible to test individual patients for the presence or absence of pneumococcal antigen - specific immune complexes and to quantify the antigenic component of the complex when present. This can be done at the earliest stage of consultation at which time the receiving physician requires to know the cause of the patient's infection. The degree or severity of the pneumococcal infection can be determined by the amount of measurable antigen contained within the immune complex. This is a new finding which is pertinent only to this invention.

In this invention the material used for binding of the immune complexes, containing the pneumococcal antigen is preferably conglutinin, a complex protein obtained from the serum of ruminants and preferentially separated from the serum of bovines, as described below. The binding of the relevant complexes to conglutinin is through the iC3b component of complement. (Laursen et al. 1994). It is clear that antibody directed against this component or its breakdown products would be a suitable substitute for conglutinin and this possibility is envisaged within the current application. Similarly, it is envisaged that immune conglutininins from human sera might be a useful substitute for bovine conglutinin, for these react with the products of complement C3 breakdown.

Conglutinin can be obtained from the serum of ruminants, preferably bovines, by using a suitable absorbent material and eluting the conglutinin from the absorbent in a high degree of purity.

Preferably, the absorbent is zymosan, which may be obtained by treating Baker's yeast (Saccharomyces cerevisiae) with 0.1M 2 - mercaptoethanol for two hours and alkylating the product with 0.02M iodoacetamide in phosphate - buffered saline solution.

Preferably, also, the conglutinin is separated from the absorbent by EDTA elution, centrifuging and dialysis. Purification of the eluted conglutinin is preferably by FPLC chromatography.

Conglutinin advantageously reacts only with a stable component of complement, iC3b, which component is incorporated into the antigen-antibody complex by the action of the complex on the complement system. It is therefore a preferred substance for the binding of immune complexes although other substances which bind to this component or derivatives of it would achieve the same result.

Conglutinin may be obtained from the sera of ruminant animals, preferably bovines, but it can be obtained from the sera of other ruminants e.g. African buffalo, water buck, Uganda Kob etc.

The conglutinin is preferably, although not exclusively, attached to a solid substrate prior to treatment with samples containing immune complexes. Many solid substrates have been shown to be useful including substrate balls of plastic, plastic strips, plastic tubes and microtitre assay plates. The preferred substrate is the microtitre assay plate, particularly those developed for maximum absorption such as Nunc - Immuno Plate, Maxisorp TM (Nunc.)

In the step of identifying the bound immune complex, reagents specific for attachment to pneumococcal antigens are used. These reagents, known as anti-antigens, are of different origin and have different specificities. Thus it is possible to use antibodies, derived from animals immunised with pneumococcal antigens and in such a use the antibody can be of any animal origin except human. It is also possible to use a range of monoclonal antibodies and there are other, non-antibody substances, such as C-reactive proteins or lectins which will react with the pneumococcal antigens contained within the immobilised immune complexes. Preferably, an antibody which reacts with a common pneumococcal antigen, common to all strains of pneumococci, e.g. C-polysaccharide antigen, such as the rabbit monospecific antibody supplied by the Serum Institut, Copenhagen, is used. However, for identification of the involvement of individual types of pneumococcus in a patient's infection, antibodies which are specific for any of the known serotypes of Streptococcus pneumoniae can be used whether these are of polyclonal or monoclonal origin.

These antibodies can be used with or without a label. In a preferred method the antibodies are not labelled and are detected at a later stage by the addition of an appropriate "second" antibody which may for example be rabbit anti-mouse antibody or sheep anti-rabbit antibody, depending on the origin of the first antibody used. The second antibodies are labelled with enzymes, radioactive isotopes or other suitable markers which can provide a quantitative measure of the amount of first antibody which has been bound in the assay. In this way, the presence or absence of reaction can be detected and the amount of antigen quantitated.

A preferred method of such measurement is to compare the "unknown" patient samples with a standard immune complex preparation. Such a standard is preferably obtained by mixing different amounts of pneumococcal antigen such as that contained in a vaccine e.g. Pneumovax, available from Pasteur Merieux MSD Ltd., Maidenhead, England and antibody of human origin which is known to contain antibody to pneumococcal antigens. Such an antibody can be obtained from persons who have been immunised with Pneumovax.

In a preferred method, known quantities of the Pneumovax antigen and known quantities of the human antibody are mixed and a quantity of fresh or suitably stored (freeze-dried, - 70°C or lower, etc.) normal serum is added to provide a source of complement. After mixing and incubating at 37°C for thirty minutes the prepared complex is assayed and provides a standard against which other estimates can be compared. The source of serum used as a complement source is preferably human, although it is possible to use complements from other species as a sensitising agent.

Embodiments of the method of this invention will now be described by way of illustration in the following examples.

### EXAMPLE 1

### PREPARATION of CONGLUTININ

A conglutinin rich bovine serum is selected by a red cell clumping test, as described on pages 172 to 175 of "The Serology of Conglutination and its relation to Disease" by Coombs et al. (Blackwell, 1961). One litre of high titre bovine serum selected in this way, of titre greater than 1 in 640, is mixed with 150g of zymosan for two hours at 4°C. The zymosan is prepared as described by Coombs et al. 1961.

After mixing, the product is then washed three times with an equal volume of veronal (barbitone) buffered saline solution and the conglutinin is eluted by adding 0.01M phosphate - buffer, containing 0.02M EDTA, for ten minutes at room temperature. The product is then centrifuged and the supernatent liquid containing the conglutinin is recovered and dialysed against 0.5M NaCl overnight at 4°C. The liquid is then purified by FPLC chromatography using the following conditions.

The purified conglutinin can be attached to any of the proscribed solid phase absorbents, by the following method which describes the procedure for microtitre plates, by way of example.

### EXAMPLE 2

### SENSITISATION of SOLID PHASE ADSORBENT

Using a conglutinin preparation obtained as described in Example 1, a Nunc-Immuno Plate, Maxisorp TM microtitre plate (Nunc) is sensitised as follows:

Each microtitre well of the plate receives 100 ul of the prepared conglutinin which is diluted 1 in 10 in coating buffer (veronal buffered saline at pH9.5). The plate is incubated, covered at 4°C overnight.

After incubation the plate is washed with a wash buffer which is veronal (barbitone) buffered saline to which has been added 0.5% Tween 20 (polyoxyethylenesorbitan monolaurate) and which has a pH of 7.4.

After washing, 150ul of 5% milk solution (obtained by dissolving any proprietary dried milk preparation in the wash buffer described above) is added to each well of the microtitre plate and incubated at 37°C for two hours, whilst the plate is covered by an occlusive membrane to prevent evaporation.

After this incubation the plate is washed three times with wash buffer using 150ul per well at each washing stage. The microtitre plate is, at this stage, ready for use and can be used immediately or, if desired, after storage for a period of time. Storage times can be up to one year if the plate is properly sensitised and occlusive covering is applied to the plate.

### EXAMPLE 3

### ASSAY of PNEUMOCOCCAL ANTIGEN SPECIFIC IMMUNE COMPLEXES

A series of standards for the assay of pneumococcal - antigen specific immune complexes is prepared by mixing pneumococcal antigens with human antibody specific for the pneumococcal antigens. A suitable source of antigens is the Pneumovax vaccine (Pasteur Merieux MSD Ltd.) and antibody to this vaccine should be from a pool of ten or more human donors who have received a course of immunisation with this vaccine. Typically the antigen and human antibody are mixed in a ratio of 1:80 respectively. After mixing, freshly obtained normal human serum (or normal human serum stored at an appropriately low temperature such as - 70°C) is added to a concentration of 10% v/v, in order to activate the complexes with complement. The normal human serum as a complement source is allowed to act at 37°C for thirty minutes.

After this time a series of dilutions of the standard is prepared (typically 1:1, 1:2, 1:2.5, 1:5 and 1:10 in a sample buffer consisting of veronal buffered saline pH 7.4 to which is added 15% foetal calf serum) and aliquots of these are included in the assay. 100ul aliquots of these standards and 100ul aliquots of patient samples, diluted 1 in 20 in the sample buffer, together with known negative human serum samples, are added to different wells of the prepared, conglutinin coated plate, which is incubated for thirty minutes at 37°C. After this the plate is washed, preferably using a specialized automatic plate washer, three times with wash buffer (veronal buffered saline pH7.4, with 0.5% Tween 20). 100ul of monospecific anti-polysaccharide C antibody diluted 1 in 100 in sample buffer is added to each well of the microtitre plate and the plate is incubated for thirty minutes at 37°C. In this example the anti-polysaccharide C antibody has no label, although in variations it can be labelled with any of the known and useful signal producing substances.

After incubation, the microtitre plate is again washed three times with wash buffer and 100ul of anti-rabbit IgG (or anti-rabbit whole Ig), labelled with alkaline phosphatase, is added to each well and incubated at 37°C for thirty minutes. The plate is finally washed three times with wash buffer and 100ul of p-nitrophenol phosphate at a concentration of 5mg in 5ml substrate diluent, is added to each well of the plate. The substrate diluent is prepared as follows. Add 2.25g glycine (C₂H₅NO₂) to 77ml distilled water and mix. Add 23ml of 1MNaOH to this and mix: Add ZnCl and MgCl to a concentration of 0.0006M, check that the pH is 10.2 and adjust to this value using 1MNaOH or 1MHCl where necessary.

After the addition of the substrate the plate is incubated at room temperature for approximately fifteen minutes after which the optical densities are read at 405 nm, preferably using an automated plate reader.

The results are as shown in Table 1, which shows the results of tests on representative patient sera and controls.

**TABLE 1**

| Sample No | OD at 405mm | Calculated unit value |
|---|---|---|
| Positive Control 1:1 | 1.785 | 100 |
| Positive Control 1:2 | 1.290 | 50 |
| Positive control 1:2.5 | 0.850 | 40 |
| Positive control 1:5 | 0.455 | 20 |
| Positive control 1:10 | 0.175 | 10 |
| 8312K | 0.120 | < 10 |
| 8539X | 0.666 | 32 |
| 8293K | 0.437 | 19.5 |
| 8303Y | 2.312 | > 100 |
| Negative control (1) | 0.151 | < 10 |
| Negative control (2) | 0.075 | < 10 |
| Negative control (3) | 0.074 | < 10 |

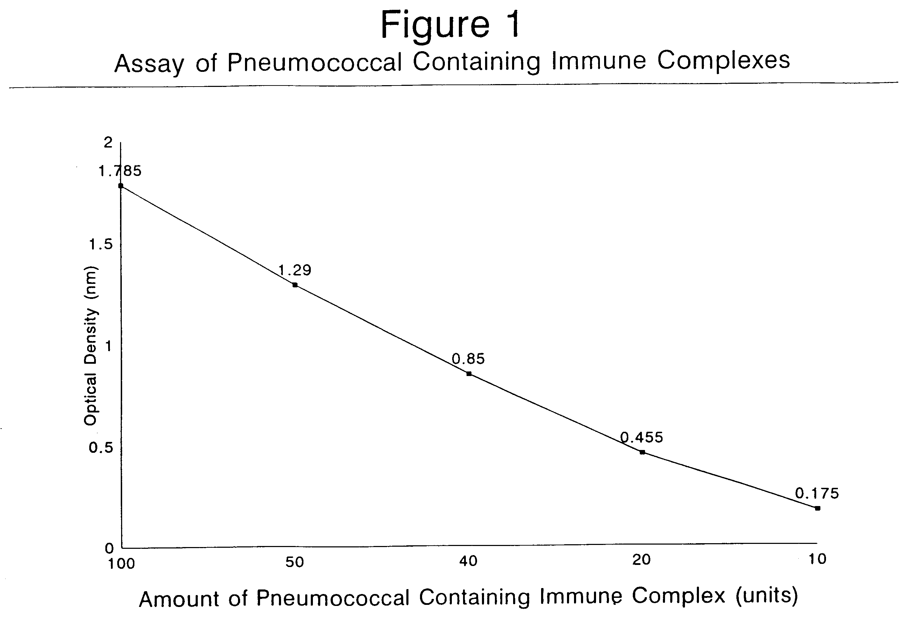

Figure 1 shows the graphical representation of the results plotted for the positive controls. Table 1 and Figure 1 are representative of the results obtained in the assay. The results for four human sera assayed by the method are shown. These represent a spectrum of the results and are not intended to describe all possible results.

In the assay described, serum 8312K is clearly negative, sera 8359X and 8293K have elevated levels and serum 8303Y has grossly elevated levels of pneumococcal containing complexes at greater than 100 nominal units per ml. To obtain a value for sera such as 8303Y it is necessary to dilute the sample further and repeat the assay.

In the results shown (Table 1) negative control (1) is a serum taken from a healthy donor known to have no respiratory disease and who probably has no pneumococcal infection. Negative control (2), is the assay as performed without the addition of monospecific rabbit anti-pneumococcal polysaccharide C antibody. Negative control 3 is the complete assay of a sample excluding the conjugate (alkaline phosphatase labelled anti-rabbit immunoglobulin) step.

Using this assay, with the controls and standards specified allows measurement of the amount of pneumococcal antigen containing immune complexes in a patient's serum. The quantitation is arbitary and based on an assumption that the prepared immune complex standard contains 100 units/ml of pneumococcal containing immune complexes. It has to be recognised that any other value for this standard could be assumed, or that an absolute value for this could be obtained by separate quantitative methods.

### References cited

1. Mellencamp et al., Infect. Imm. vol 55(8): pp 1737-1742 (1987)
2. Parratt, D US Patent 4,548,909 (1985) "A Method of Diagnosis".
3. Manca et al., Trans Roy Soc. Trop. Med. Ayg. vol 82, pp.254 - 257 (1988)
4. Bo-Hai W and Shu-Rong y, Exp. Mol. Path. vol 47, pp 175-184 (1987)
5. Laursen et al., Immunology, vol 81, pp 648-654 (1994)

## Claims

1. A method of diagnosis of pneumococcal infection in man by the detection and quantitation of pneumococcal-specific antigen containing immune complexes.

2. A method according to claim 1 whereby the detection and quantitation of the pneumococcal-specific immune complexes is by the binding of the complexes to conglutinin or to substances with properties similar to conglutinin whether by solid phase or liquid phase attachment.

3. A method according to claims 1 and 2 whereby the detection and measurement of the pneumococcal antigen contained in the bound complex is by pneumococcal species specific or pneumococcal serotype specific antigen recognition.

4. A method according to claims 1, 2 and 3 whereby the detection and measurement of the pneumococccal antigen is by the use of a second antibody or similarly reactive substance to which a quantifiable label is attached.

5. A method according to claims 1, 2, 3 and 4 which relates all such measurements to a series of antigen-specific immune complex standards obtained from the reaction of pneumococcal antigens with human antibody to these antigens, which complexes have been sensitised with human complement from a suitable normal donor, who is free of pneumococcal disease or by sensitisation with complement from a different animal species, similarly free from pneumococcal disease.

6. A method which according to claims 1, 2, 3, 4 and 5 binds immune complexes in the patient's serum via the iC3b subunit of complement.

7. A method according to claims 1, 2, 3, 4, 5 and 6 which, although preferably using conglutinin, can use other proteins or substances to bind complexes through the iC3b subunit of complement.

8. A method which according to claims 1 - 7 can utilise any suitable preparation of pneumococcal antigen or antigens in combination with antibody of human or animal origin to provide a standard or series of standards against which to measure samples with unknown values from patients' sera.

9. A method according to claims 1 - 8 which uses any antibody or substance capable of reacting with antigens derived from all or the most common pneumococcal strains as a detecting agent for the bound immune complexes.

10. A method according to claims 1 - 9 which uses a detecting antibody or substance which is labelled in some way, so as to provide a quantifiable signal.

11. A method according to claims 1 - 10 which includes any signal - derived method such as radioactivity, enzyme action, photon production and other methods which provide a measure of binding of one antibody or substance to an antigen or other pneumococcal derived substance, in order to provide a quantifiable measure of the amount of pneumococcal antigen present in the immune complexes of patients with pneumococcal induced disease.

12. A method according to claims 1 - 11 which can be used for diagnosis by testing samples taken from the patient at any time during the onset or course of the infection.

13. A method according to claim 2 which uses a purified preparation of conglutinin obtained by FPLC chromatography.

14. A method according to claim 2 whereby the binding of conglutinin or according to claim 7, other suitable binding substances, uses a method of attachment to solid phase reactants, which involves the use of

15. A method according to claim 5 whereby the human or animal serum used as a source of complement to sensitise the immune complex standards can be fresh or stored freeze-dried or at temperatures below -70°C.
